# EUROPEAN PATENT SPECIFICATION

(11) **EP 2 606 892 B1**
(45) Date of publication and mention of the grant of the patent: **24.08.2016**
(21) Application number: 12196726.9
(22) Date of filing: 12.12.2012
(51) Int. Cl.: A61K 31/46, A61K 47/10, A61K 47/12, A61K 9/00, A61K 9/12, A61M 11/04, A61M 15/00, A61P 11/08

(54) **AN INHALER COMPRISING A TIOTROPIUM-CONTAINING-COMPOSITION**
INHALATOR ENTHALTEND EINE TIOTROPIUM ZUBEREITUNG
INHALATEUR COMPRENANT UNE FORMULATION COMPRENANT DU TIOTROPIUM

(30) Priority: 19.12.2011 US 201161577314 P; 13.01.2012 GB 201200504
(43) Date of publication of application: 26.06.2013
(73) Proprietor: Teva Branded Pharmaceutical Products R&D, Inc., Frazer PA 19355 (US)
(72) Inventor: Zeng, Xian-Ming, Miami, 33169-5043 (US); Fenlon, Derek, Co. Wexford (IE)
(74) Representative: Gillard, Richard Edward

(56) References cited:
- EP-A1- 2 201 934
- WO-A2-01/93933
- GB-A- 2 264 238
- US-A1- 2004 018 153

## Description

The present invention relates to an inhaler and more specifically to an inhaler for the pulmonary administration of tiotropium.

Tiotropium is an anticholinergic agent and is indicated as a maintenance bronchodilator treatment to relieve symptoms of patients with chronic obstructive pulmonary disease (COPD). Tiotropium is marketed as Spiriva® in the form of an inhalation powder or solution for inhalation.

Tiotropium contains a quaternary ammonium cation and is typically used as the bromide salt which has the following structure:

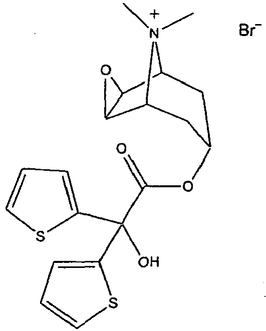

Anticholinergic agents are active pharmaceutical agents which reduce the bronchoconstrictive effects of acetylcholine. Anticholinergics are typically reversible competitive inhibitors of one of the two types of acetylcholine receptors: antimuscarinic agents which operate on the muscarinic acetylcholine receptors and antinicotinic agents which operate on the nicotinic acetylcholine receptors. Tiotropium is an example of an antimuscarinic agent. It blocks the M₁-M₃ subtypes of muscarinic receptors in the bronchial smooth musculature. While the M₁ and M₃ receptors mediate bronchoconstriction, M₂ receptors inhibit the release of acetylcholine and therefore provide feedback inhibition. Blocking of the M₂ receptor with a non-selective antimuscarinic agent can therefore lead to an increase in acetylcholine release, which may overcome the blockade of other muscarinic receptors. Tiotropium dissociates more rapidly from the M₂ receptor than from the M₁ and M₃ receptors and is therefore selectively antagonistic for the receptors that mediate bronchoconstriction (i.e. it shows kinetic selectivity).
Tiotropium is thus a long-acting, specific muscarinic receptor antagonist. The high and slow receptor dissociation of tiotropium correlate with significant and long-acting bronchodilation in patients with COPD. This extremely slow dissociation from the provides a half life of greater than 36 h. In contrast, previously available antagonists, such as atropine and ipratropium, are nonselective muscarinic block M₁-M₃ receptors. The longer activity of tiotropium has the effect that tiotropium may be dosed once daily whereas, say, ipratropium (Atrovent®) requires dosing four day (see M.C. Durham "Tiotropium (Spiriva): a once-daily inhaled anticholinergic for chronic obstructive pulmonary disease" BUMC Proceedings, 2004,17, 366-373 and Rennard, The Lancet, 2004, 364, 791-802).

Whilst the high potency and long-acting duration of action result in tiotropium being an extremely effective bronchodilator, it carries with it the significant risk of undesirable side effects if tiotropium is inadvertently delivered to the eye. The reason tiotropium presents a particular risk is that muscarinic receptors regulate a number of important physiological processes in the eye. In particular, topical delivery to the eye may result in dilation of the pupils (mydriasis) and, in some instances, accommodation paralysis (cycloplegia).
This drawback has been widely reported. S.I. Rennard, The Lancet, 2004, 364, 791-802 discusses the long half life of tiotropium (greater than 36 h) and warns at page 796 that "Local effects can occur if directly sprayed in the eye." The Nurse's Drug Handbook, Tenth Edition, Ed. A. Sibley, Jones & Bartlett Learning, 2011 refers to the risk of mydriasis and cycloplegia if tiotropium is inadvertently administered to the eye. The summary of product characteristics (SmPC) for the commercial product presently on the market, the Spiriva® HandiHaler®, warns that "Patients should be cautioned to avoid getting the drug powder into their eyes. They should be advised that this may result in precipitation or worsening of narrow-angle glaucoma, eye pain or discomfort, temporary blurring of vision, visual halos or coloured images in association with red eyes from conjunctival congestion and corneal oedema." Similar warnings are provided for the soft-mist Spiriva® Respimat® product.
The most significant risk of inadvertent administration to the eye comes with the use of a pressurised metered dose inhaler (pMDI). The pMDI is the most preferred approach for the pulmonary administration of medicaments outside of the emergency room. Typically patient compliance is greater with a pMDI as they tend to be easier to use. Moreover, the DPI and soft-mist inhalers suffer from the drawback that only a small portion of the powdered active ingredient is actually inhaled into the lungs. However, the pMDI is the most likely type of delivery device to lead to accidental delivery of the medicament to the eye.
It is noteworthy that the two products on the market are a dry powder inhaler (HandiHaler®) which is not prone to accidental delivery to the eye because the inhaler is not pressurised, and a soft-mist inhaler (Respimat®) which produces a low pressure mist which is less likely that a pMDI to be inadvertently spayed into the eye.

Moreover, Anticholinergic Agents in the Upper and Lower Airway, Ed. S.L. Spector, Marcel Dekker, Inc., 2005 explains in the discussion of tiotropium at page 37 that "Side effects do not appear to be a problem at doses that are useful clinically, although it will be important to protect against eye contact; a dry powder inhaler formation rather than a metered dose inhaler may be the most appropriate."

There are therefore sound technical reasons against using pMDIs for the delivery of tiotropium salts. There remains, therefore, a need in the art for an effective approach for administering tiotropium salts to the lungs without risking inadvertent delivery to the eyes.

EP 2 201 934 discloses a pressurized metered dose inhaler comprising an aerosol can fitted with a metering valve comprising sealing ring/s and/or gasket/s, which is/are in contact with the formulation, made of a butyl or halo-butyl rubber wherein the aerosol can contains a medicinal aerosol solution formulation comprising a tiotropium salt, a hydrofluorocarbon propellant, one or more co-solvents, and a mineral acid.

Accordingly, the present invention provides a pressurised metered dose inhaler comprising a canister, wherein the canister contains a formulation comprising a tiotropium salt and an HFA propellant, wherein the inhaler is an inhalation-actuated inhaler comprising a resiliently deformable member for applying a preload capable of actuating the internal valve of the canister to release a metered dose of the formulation from the canister, a mechanism for applying a resisting pneumatic force capable of preventing actuation of the aerosol valve and an inhalation-actuated release device capable of releasing the resisting pneumatic force to allow the preload to actuate the aerosol valve and allow the metered dose of the formulation to be dispensed.

Thus, by using an inhalation-actuated inhaler (also known as breath-actuated inhaler), one can obtain the benefits of administration using a pressurised propellant system without the significant risks of inadvertent administration to the eye.

The present invention will now be described with reference to the accompanying drawings, in which:
Fig. 1 shows a section view of an inhalation-actuated inhaler according to the present invention;
Fig. 2 shows an enlarged view of a diaphragm for use in the inhaler shown in Fig. 1; and
Fig. 3 shows an enlarged view of the diaphragm in position in (a) a pre-actuated and (b) an actuated state.

### The inhaler

Standard pMDIs are well known in the art (see, for example, Drug Delivery to the Respiratory Tract, Eds. D. Ganderton and T. Jones, VCH Publishers, 1987, pages 87-88, or Pharmaceutics - The Science of Dosage Form Design, Second Edition, Ed. M.E. Aulton, Churchill Livingstone, 2002, page 476 et seq.). pMDIs typically have a medicament-containing canister located in an actuator housing having a mouthpiece. The canister is usually formed from an aluminium cup having a crimped lid which carries a metering valve assembly. The metering valve assembly is provided with a protruding valve stem which is inserted as a push fit into a stem block in the actuator housing.

To actuate, the user applies a compressive force to the closed end of the canister. The internal components of the metering valve assembly are spring loaded so that, compressive force of 15 to 30 N (usually around 20 N) is required to activate the response to this compressive force, the canister moves axially with respect to the by an amount varying between about 2 and 4 mm. This degree of axial movement is to actuate the metering valve and cause a metered quantity of the formulation to be through the valve stem. This is then released into the mouthpiece via an orifice in the dispensing nozzle of the stem block. A user inhaling through the mouthpiece of the this point will thus receive a dose of the active ingredient.

The inhalation-actuated inhaler operates on a similar principle, but the action of inhalation actuates the inhaler without the user having to apply the compressive force to the canister manually. Suitable breath-actuated inhalers are known in the art. See, for example, WO 92/09323, GB 2 264 238 and WO 01/93933.

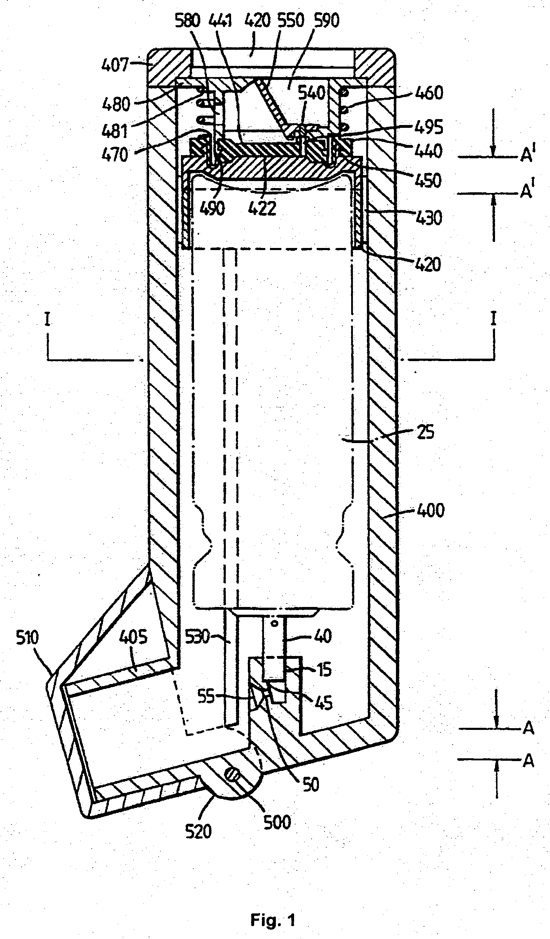

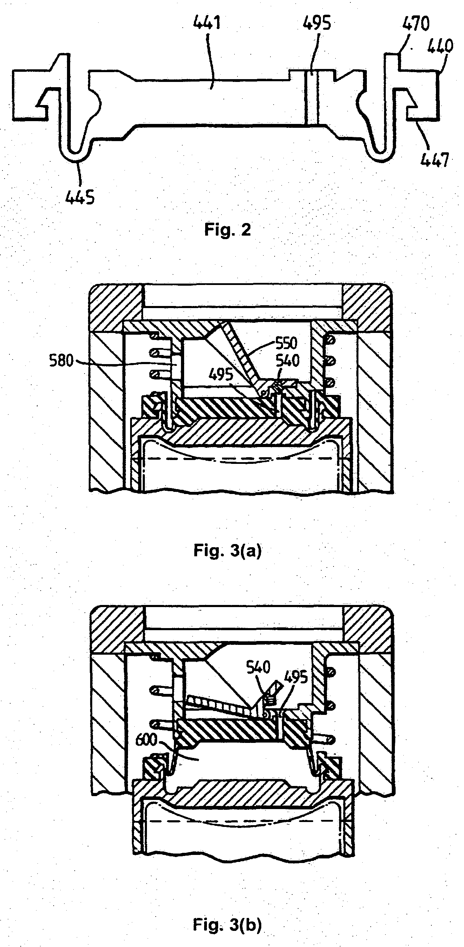

Fig. 1 shows an inhaler having a main body 400 which is generally cylindrical in cross section, with a mouthpiece section 405 at one end and an end cap 407 housing air inlets 420 at the other end. A known type of aerosol dispensing canister 25 of generally cylindrical shape is housed within the main body of the inhaler. The aerosol dispensing canister has a stem 40 which contains an aerosol dispensing valve (not shown). The bore 15 is such that it forms an air tight seal on the stem 40 of the aerosol dispensing canister 25. A shoulder 45 limits and locates the position of the stem 40, which in turn locates the aerosol dispensing canister 25 in position in the main body 400. A passage 50 extends from the bore 15, continuing from the shoulder 45 to interconnect with a dispensing nozzle 55.
The opposite end of the dispensing canister is contained within a sleeve 420 of similar cross section to the main body 400. The longitudinal axis of both the sleeve 420 and main body 400 is generally coaxial. The sleeve is in loose sliding contact with the inner wall of the main body and may include several rebated grooves 430 in its walls to allow free passage of air in the main body past the sleeve. The sleeve 420 may be held in place by connection with a diaphragm 440 held in connection with the top of the main body 400, as will now be described. Thus, the sleeve 420 effectively hangs from the top of the main body.
One end of an e.g. moulded flexible diaphragm 440 (as shown alone in Fig. 2) comprising a rigid disc-like section 441, a flexible generally cylindrical wall section 445 and a stiffer connector section 447, is fitted around a purpose-made groove 450 in the sleeve, e.g. by snap-fitting. A further moulded lip 470 on the diaphragm provides a snug fit for one end of a compression spring 460. The compression spring is thus located and free to act on the sleeve. The other end of the compression spring is located by an annular shoulder 481 in a predominantly cylindrical flanged insert 480 housed in the top section of the main body 400. This insert includes a groove 490 into which the disc-like section 441 of the flexible diaphragm 440 is snap-fitted.

The joint between the diaphragm connector section 447 and inner sleeve groove 450 is arranged to be air tight and the shape of the top surface of the sleeve 422 to conform to the internal shape of the diaphragm such that in the rest position of the inhaler the two surfaces are in close proximity, and the enclosed space between them very small.

The cylindrical insert 480 is retained in place by the end cap 407 fitted into the main body of the inhaler. This forms a chamber 590 between the air inlet slots 420 and the rigid part 441 of the diaphragm. The chamber is provided with one or more air pathways 580 such that air may pass from the air inlet slots 420 to the mouthpiece 405. The rigid disc-like section 441 of the diaphragm also includes a small valve port 495 which is normally covered by a valve seal (flap) 540 housed in a vane 550 pivotally connected to the insert 480.
The vane 550 in its rest position divides the chamber 590 between the air inlets 420 and the air pathways 580 that link to the mouthpiece such that it may move from its rest position by means of a pressure drop between the air inlets and the mouthpiece. On movement of the vane to the actuated position the valve seal (flap) 540 is sufficiently moved to open the valve port 495. (The vane 550 may be biased closed by a light spring flexure, a weight or a magnet not shown.)

As shown in Fig. 1, the end of the main body having a pivot 500 has a recess adapted to receive a cam 520 integral with a dust cap 510 operating on the pivot. The recess further includes a passage communicating with a similar passage moulded into the internal wall of the main body 400. A cam-follower 530 extending from the lower edge of the inner sleeve 420 acts on the cam such that when the dust cap is in the closed position the inner sleeve is forced by the cam-follower to its uppermost position.

When the dust cap is rotated to its open position the cam profile is such that the cam-follower is free to move downwards by an amount sufficient to allow actuation of the inhaler.
In its rest position the dust cap 510 is closed, the cam-follower 530 restrains the inner sleeve 420 in its uppermost position such that the enclosed space trapped between the diaphragm 440 and the top surface 422 of the inner sleeve is at a minimum and the spring 460 is compressed. The valve port 495 is closed by the valve seal (flap) 540 and the sleeve 420 is clear of the top of the aerosol can 25 which is thus unloaded.

The dust cap is opened rotating the integral cam 520 allowing the cam-follower 530 to amount AA. The inner sleeve is forced downwards under the action of the spring 460. inner sleeve moves downwards the enclosed volume between the diaphragm 440 and sleeve is increased by a linear equivalent amount A'A', less than or equal to AA. Since valve port 495 is closed this creates a low pressure volume or near vacuum in the

(Fig. 2). The effect of the pressure differential between the enclosed volume 600 and atmospheric pressure is such that the inner sleeve tends to resist the action of the the inner sleeve moves downwards it contacts the aerosol can 25 and begins the aerosol valve (not shown).

Downward movement of the inner sleeve will continue until there is a balance of forces between the compressive force in the spring 460 and resisting forces created by the pressure differential and compression of the aerosol valve. The geometry of the inhaler is arranged such that this balance occurs before the aerosol valve has been sufficiently compressed to actuate it.

A typical aerosol requires about 20N force to actuate. The spring 460 should accordingly provide a greater force, preferably 10% to 50% greater.

It may also be possible to arrange for the balance of forces to take place before the inner sleeve has contacted the aerosol can, such that the spring force is balanced by the resisting force produced on the inner sleeve by virtue of the pressure differential.

On inhalation by the patient through the mouthpiece 405, a small pressure differential is created across the vane 550 which is pivoted towards one end. The pressure differential causes the vane to move from the rest position to the actuated position. The vane and design of the air passageway 580 in the chamber 590 are such that in the actuated position air can flow freely from the air inlets 420 to the patient.

The movement of the vane 550 causes the valve seal (flap) 540 to be moved out of a sealing position with the valve port 495. Opening the valve port allows air into the gap 600 between the diaphragm and inner sleeve such that the enclosed space reaches atmospheric pressure. This causes an imbalance of forces acting on the sleeve 420 and canister 25. The sleeve and canister are thus forced downwards by the spring 460 resulting in the release of a measured dose of medicament through the dispensing nozzle 55 and into the mouthpiece at the same time as the patient breathes in. Thus the patient inhales air with a metered dose of medicament.

After the inhalation of the dose by the patient, the dust cap 510 is returned to its closed position. This rotates the cam 520 and causes the cam-follower 530 to be forced This in turn acts on the inner sleeve 420 moving it upwards to compress the spring 460 close the gap 600 between the diaphragm and inner sleeve top surface 422. This forces out of the enclosed space 600 which escapes through the valve port 495 lifting the (flap) 540. Since the valve seal (flap) is only lightly biased to its closed position it little resistance to air flow out of the enclosed space. The aerosol can is free to return rest position under the action of its own aerosol valve spring.

In use the patient loads the aerosol dispensing canister into the main body. The aerosol canister may be loaded by providing a coarse threaded screw in the main body 400, for example about the line I-I. When part of the main body 400 has been unscrewed, the aerosol can be inserted. The main body 400 can then be replaced locating the inner sleeve over the top end of the can, and the inhaler is ready for use. As described previously, the inhaler could be manufactured as a sealed unit.

The inhaler may be provided with means to provide a regulated air flow to the user or inhaler. Thus a sonic device, e.g. a reed, may be provided which sounds when the inspired air flow is greater than a pre-set level, e.g. above 30 to 50 litres per minute. The sonic device may be located in the mouthpiece 95 or below the air inlet 420. The sound produced warns the patient to breathe at a lower rate.

The inhaler may also be provided with a means such that it will not operate below a certain pre-determined air flow rate, e.g. 10 to 30 litres per minute. In one embodiment the vane 550 or 110 will be biased by a spring such that the predetermined minimum air flow is necessary for it to move to its actuated position and enable the valve seal to open.
Accordingly, in a preferred embodiment, the inhaler of the present invention comprises a resiliently deformable member 460 for applying a preload capable of actuating the internal valve of the canister to release a metered dose of the formulation from the canister, a mechanism 440,495,540 for applying a resisting pneumatic force capable of preventing actuation of the aerosol valve 40 and an inhalation-actuated release device 540 capable of releasing the resisting pneumatic force to allow the preload to actuate the aerosol valve 40 and allow the metered dose of the formulation to be dispensed

The main body of a inhaler is preferably manufactured from a plastic such as polypropylene, acetal or moulded polystyrene. It may however be manufactured from metal or another suitable material.

### The formulation

The formulation of the present invention may be a suspension formulation or a solution formulation.

As tiotropium salts are generally insoluble in HFA propellants, without any further a tiotropium salt will form a suspension formulation. A suitable suspension in the present invention is set out in US 2004/018153. This document discloses bromide monohydrate in HFA 134a or 227 formulated as a suspension. The optionally contain other excipients to stabilise the suspension, such as a surfactant, surfactants are Polysorbate 20, Polysorbate 80, Myvacet 9-45, Myvacet 9-08, isopropylmyristate, oleic acid, propyleneglycol, polyethyleneglycol, Brij, ethyl oleate, trioleate, glyceryl monolaurate, glyceryl monooleate, glyceryl monostearate, glyceryl monoricinoleate, cetylalcohol, sterylalcohol, cetylpyridinium chloride or combinations

In contrast, the solution formulation is a single homogeneous phase. The tiotropium salt is thus dissolved in the propellant and typically a co-solvent is present to solubilise the active ingredient. As the active ingredient is dissolved in the propellant system, this approach avoids problems such as potential blockage of the pMDI dispensing nozzle orifice, physical instability of the suspended particles and the requirement to use suspending agents such as surfactants. Solution formulations are also easier to manufacture.
A solution formulation does not require the presence of surfactants (which are used to stabilise suspended particles of the active ingredient in a suspension formulation). Accordingly, it is not necessary to add surfactant to the formulation and hence the solution formulation of the present invention is preferably substantially free of surfactant (e.g. the solution formulation contains less than 0.0001% by weight of surfactant based on the total weight of the formulation).

The solution formulation of the present invention preferably further comprises a first co-solvent, more preferably ethanol. The ethanol is preferably dehydrated ethanol according to the USP. The ethanol is preferably included at 12-20%, more preferably 12-15%, by weight based on the total weight of the formulation.

The solution formulation may also contain water, preferably purified water, according to the USP. The water is preferably present at less than 1.00%, more preferably 0.15-0.75%, most preferably 0.30-0.60%, by weight based on the total weight of the formulation.
The solution formulation preferably further comprises a mineral acid or an organic acid. The acid helps prevent chemical degradation of the tiotropium salt in the presence of the co-solvent(s). The most preferred acid is citric acid, preferably anhydrous citric acid according to the USP. The amount of acid is probably less than 0.5%, more preferably 0.05-0.10%, most preferably 0.05-0.08%, by weight based on the total weight of the formulation.
Two suitable formulations are as follows:

| **Ingredient** | **Concentration (% w/w)** | **Concentration (% w/w)** |
|---|---|---|
| Tiotropium bromide | 0.01107 | 0.01107 |
| Ethanol, anhydrous, EP | 20.0 | 20.0 |
| Citric acid, EP | 0.06 | 0.06 |
| Purified water, EP | 0.50 | 0.50 |
| Glycerol EP | 1.50 | - |
| HFA 134a | 77.93 | 79.43 |
| **Total** | 100.0 | **100.0** |

They deliver 5.25 µg tiotropium as 6.3 µg tiotropium bromide (ex-valve) per actuation from a 50 µL valve.

As described hereinabove, on actuation of the inhaler, a metered dose of the formulation is released from the inhaler. The metered dose of the formulation passes through the valve stem and stem block where it is discharged via an orifice in the dispensing nozzle of the stem block into the mouthpiece and hence to the patient. On release, the propellant rapidly evaporates leaving the active ingredient dissolved in small droplets of ethanol and water which will in turn evaporate to some extent. The particle size of the droplets will depend on a number of factors, including the precise amounts of ethanol and water used, the size of the orifice in the dispensing nozzle, the spray force, the plume geometry, etc. Typically, however, the droplets will be less than 5 microns in diameter. For some applications, the droplet sizes will be too small for optimal lung deposition. In such cases, a second co-solvent having a higher boiling point than the first co-solvent may be used. For example, the first co-solvent may be ethanol and the second co-solvent may be glycerol. Glycerol is less volatile than ethanol and hence experiences less evaporation on actuation, thereby providing larger droplets (by larger is meant that they have a higher mass median aerodynamic as measured by an NGI). Accordingly, in a preferred embodiment, the solution formulation of the present invention further comprises glycerol..
The present invention is applicable to tiotropium salts generally, but preferably the present formulation contains tiotropium bromide which is the most commonly used salt and the salt presently on the market. The formulations set out hereinabove are particularly, but not exclusively, designed for use with tiotropium bromide as the tiotropium salt.

The amount of tiotropium salt present will vary depending on the dose of tiotropium required for the particular product. Typically, the tiotropium salt (preferably the present in an amount to provide 2-10 micrograms of tiotropium base, ex valve, per That is, the amount of free base equivalent in the metered dose as measured as it valve. This corresponds to a preferred amount of tiotropium bromide of 0.00422-wt%.

The formulation also contains a hydrofluoroalkane (HFA) propellant. Such propellants are well known in the art. The preferred HFAs of the present invention are HFA 134a and/or HFA 227. Preferably HFA 134a is used.

## Claims

1. A pressurised metered dose inhaler comprising a canister (25), wherein the canister (25) contains a formulation comprising a tiotropium salt and an HFA propellant, wherein the inhaler is an inhalation-actuated inhaler comprising a resiliently deformable member (460) for applying a preload capable of actuating the internal valve (40) of the canister (25) to release a metered dose of the formulation from the canister (25), a mechanism (440,495,540) for applying a resisting pneumatic force capable of preventing actuation of the aerosol valve (40) and an inhalation-actuated release device (540) capable of releasing the resisting pneumatic force to allow the preload to actuate the aerosol valve (40) and allow the metered dose of the formulation to be dispensed.

2. An inhaler as claimed in claim 1, wherein the tiotropium salt is tiotropium bromide.

3. An inhaler as claimed in claims 1 or 2, wherein the HFA propellant is HFA 134a and/or HFA 227.

4. An inhaler as claimed in any preceding claim, wherein the formulation is a suspension formulation.

5. An inhaler as claimed in any of claims 1 to 3, wherein the formulation is a solution formulation.

6. An inhaler as claimed in claim 5, wherein the solution formulation further comprises a first co-solvent.

7. An inhaler as claimed in claim 6, wherein the solution formulation further comprises a second co-solvent having a higher boiling point than the first co-solvent.

8. An inhaler as claimed in any of claims 5 to 7 wherein the solution formulation further comprises a mineral acid or an organic acid.

9. An inhaler as claimed in any of claims 5 to 8, wherein the solution formulation comprises a tiotropium salt, ethanol, water, citric acid, HFA 134a and optionally glycerol.

## Patentansprüche

1. Unter Druck stehender Dosisinhalator, einen Behälter (25) umfassend, wobei der Behälter (25) eine Rezeptur enthält, die ein Tiotropiumsalz und ein HFA-Treibmittel umfasst, wobei der Inhalator ein durch Inhalation aktivierter Inhalator ist, der ein elastisch verformbares Element (460) zum Anwenden einer für das Aktivieren des innenliegenden Ventils (40) des Behälters (25) geeignete Vorspannung (25), um eine gemessene Dosis der Rezeptur aus dem Behälter (25) abzugeben, umfasst, ferner ein Mechanismus (440, 495, 540) zum Anwenden einer resistenten pneumatischen Kraft, die in der Lage ist, das Aktivieren des Aerosolventils (40) zu verhindern, und eine durch Inhalation aktivierte Abgabevorrichtung (540), die in der Lage ist, die resistente pneumatische Kraft so freizusetzen, dass die Vorspannung das Aerosol-Ventil (40) aktivieren kann und die gemessene Dosis der Rezeptur abgegeben werden kann.

2. Inhalator nach Anspruch 1, wobei es sich bei dem Tiotropiumsalz um Tiotropiumbromid handelt.

3. Inhalator nach Anspruch 1 oder 2, wobei es sich bei dem HFA-Treibstoff um HFA 134a und/oder HFA 227 handelt.

4. Inhalator nach einem der vorstehenden Ansprüche, wobei es sich bei der Rezeptur um eine Suspensionsrezeptur handelt.

5. Inhalator nach einem der Ansprüche 1 bis 3, wobei es sich bei der Rezeptur um eine Lösungsrezeptur handelt.

6. Inhalator nach Anspruch 5, wobei die Lösungsrezeptur ferner ein erstes Zusatzlösungsmittel umfasst.

7. Inhalator nach Anspruch 6, wobei die Lösungsrezeptur ferner ein zweites Zusatzlösungsmittel umfasst, das einen höheren Siedepunkt aufweist als das erste Lösungsmittel.

8. Inhalator nach einem der Ansprüche 5 bis 7, wobei die Lösungsrezeptur ferner eine Mineralsäure oder eine organische Säure umfasst.

9. Inhalator nach einem der Ansprüche 5 bis 8, wobei die Lösungsrezeptur ein Tropiumsalz, Ethanol, Wasser, Zitronensäure, HFA 134a und optional Glycerin umfasst.

## Revendications

1. Inhalateur doseur pressurisé comprenant un réservoir (25), ledit réservoir (25) contenant une formulation comprenant un sel de tiotropium et un propulseur HFA, ledit inhalateur étant un inhalateur actionné par inhalation qui comprend un élément élastiquement déformable (460) destiné à appliquer une précharge capable d'actionner la valve interne (40) du récipient (25) pour libérer une dose mesurée de la formulation provenant du récipient (25), un mécanisme (440, 495, 540) destiné à appliquer une force pneumatique résistante capable d'empêcher l'actionnement de la valve aérosol (40) et un dispositif de relâchement actionné par inhalation (540) capable de relâcher la force pneumatique résistante pour permettre à la précharge d'actionner la valve aérosol (40) et de permettre la distribution de la dose mesurée de la formulation.

2. Inhalateur selon la revendication 1, ledit sel de tiotropium étant le bromure de tiotropium.

3. Inhalateur selon la revendication 1 ou 2, ledit propulseur HFA étant le HFA 134a et/ou le HFA 227.

4. Inhalateur selon l'une quelconque des revendications précédentes, ladite formulation étant une formulation en suspension.

5. Inhalateur selon l'une quelconque des revendications 1 à 3, ladite formulation étant une formulation en solution.

6. Inhalateur selon la revendication 5, ladite formulation en solution comprenant en outre un premier co-solvant.

7. Inhalateur selon la revendication 6, ladite formulation en solution comprenant en outre un second co-solvant ayant un point d'ébullition supérieur à celui du premier co-solvant.

8. Inhalateur selon l'une quelconque des revendications 5 à 7, ladite formulation en solution comprenant en outre un acide minéral ou un acide organique.

9. Inhalateur selon l'une quelconque des revendications 5 à 8, ladite formulation en solution comprenant un sel de tiotropium, de l'éthanol, de l'eau, de l'acide citrique, du HFA 134a et éventuellement du glycérol.
